**Europäisches Patentamt**

(19) **European Patent Office**

**Office européen des brevets**

(11) Publication number : **0 331 283 B1**

(12) # EUROPEAN PATENT SPECIFICATION

(45) Date of publication of patent specification :
28.04.93 Bulletin 93/17

(51) Int. Cl.⁵ : **B01J 20/06,** B01J 39/12,
G01N 30/48, C01G 25/02

(21) Application number : 89300824.3

(22) Date of filing : 27.01.89

(54) High stability porous zirconium oxide spherules.

(30) Priority : **03.02.88 US 151819**

(43) Date of publication of application :
06.09.89 Bulletin 89/36

(45) Publication of the grant of the patent :
28.04.93 Bulletin 93/17

(84) Designated Contracting States :
**CH DE FR GB IT LI SE**

(56) References cited :
EP-A- 0 162 716
EP-A- 0 216 730
EP-A- 0 273 756
EP-A- 0 280 673
DE-A- 3 440 018

(73) Proprietor : **REGENTS OF THE UNIVERSITY OF MINNESOTA**
**Morrill Hall, 100 Church Street Southeast**
**Minneapolis, Minnesota 55455 (US)**

(72) Inventor : **Carr, Peter W.**
**1569 East River Terrace**
**Minneapolis Minnesota 55414 (US)**
Inventor : **Rigney, Martin O.**
**1193 Charles Avenue**
**St. Paul Minnesota 55104 (US)**
Inventor : **Funkenbusch, Eric F.**
**2300 Lake Ridge Drive**
**White Bear Lake Minnesota 55110 (US)**
Inventor : **Coleman, Patrick L.**
**4101 Vincent Avenue**
**Minneapolis Minnesota 55410 (US)**
Inventor : **Hanggi, Douglas A.**
**1929 Ebertz Court**
**St. Paul Minnesota 55119 (US)**

(74) Representative : **Baillie, Iain Cameron**
**c/o Ladas & Parry, Altheimer Eck 2**
**W-8000 München 2 (DE)**

## Description

Currently-known inorganic chromatography supports comprising particulate silica ($SiO_2$) or alumina ($Al_2O_3$) are stable over pH ranges of 1-8 and 3-12, respectively. The solubilization of $SiO_2$ and $Al_2O_3$ at pHs outside of these ranges results in deterioration of these supports and contamination of the resultant chromatographed and separated products with silicon- or aluminum-containing species. Methods of improving the alkaline stability of particulate $SiO_2$ by cladding the surface with a more base-stable metal oxide such as zirconium oxide ($ZrO_2$) have been disclosed in U.S. Patent Nos. 4,648,975 and 4,600,646. This cladding is disclosed to increase the upper pH limit at which these supports, also referred to as packings, can be used to 11 and 9.5, respectively. However, these packings still lack adequate stability to allow them to be sterilized and cleaned in, for example, 0.1 N aqueous sodium hydroxide (NaOH, pH=13).

Use of porous spherical $ZrO_2$ particles on a thin layer chromatography plate has been disclosed in U.S. Patent No. 4,138,336, a process for the preparation of porous $ZrO_2$ microspheres is taught in U.S. patent No. 4,010,242, and chromatographic use of these particles is taught in U.S. patent No. 3,782,075. The microspheres are prepared by a process in which colloidal metal oxide particles are mixed with a polymerizable organic material and coacervated into spherical particles by initiating polymerization of the organic material. This is a time consuming, batch process which requires the addition of organic material which is pyrolized and hence lost.

U.S. Patent No. 3,862,908 discloses microspheres of urania and other metal oxides, however, these particles are fired to near full density, have reduced surface areas and therefore, would not be attractive for chromatographic uses.

U.S. Patent No. 3,892,580 discloses a process for preparing porous bodies of $ZrO_2$. This process requires the use of a binder to react with the oxide particles during preparation. This binder is subsequently decomposed by pyrolysis and therefore lost. The bodies produced by this process are not spherical, would pack unevenly, may cause increased column pressure, and are therefore not attractive for chromatographic uses.

U.S. Patent No. 4,389,385 teaches the preparation of porous gels and ceramic materials by dispersing solid particles of an inorganic substance produced by a vapor phase condensation method in a liquid to form a sol. The sol contains colloidal particles which are aggregates of the primary particles. The sol is dried to produce a porous gel of greater than 70% by volume porosity.

The majority of separations employing high pressure liquid chromatographie (HPLC) are performed in the so-called reversed-phase mode. In this mode, the column-packing material is referred to as stationary phase. The most commonly used stationary phases feature a non-polar ligand (e.g., octane or octadecane) covalently-bound to a porous silica particle through a siloxane bond (Si-O-Si) to render the surface hydrophobic. Although these silica-based bonded phases are very useful for a wide range of applications in reversed-phase HPLC, their use is strictly limited to the pH range of between 2 and 8, due to the hydrolytic instability of both the silica support particle and the siloxane bond used to "anchor" the non-polar active group. Thus, the production of a pH-stable reversed-phase support material must involve the development of both a stable, controlled porosity, high surface area support material and a method for rendering the surface permanently hydrophobic.

The eluent, also referred to as the mobile phase, used to elute the various components from the stationary phase is relatively polar, e.g., an aqueous buffer or a mixture of water and an organic solvent, e.g., aqueous alcohol. Its polarity can be changed by increasing the concentration of the less polar liquid in the mobile phase, a technique known in the art.

Thus relative to the use of $ZrO_2$-clad silica, a more promising approach to developing a highly stable reversed-phase support, involves replacing the silica with an alternative inorganic material, such as alumina. Although it has been demonstrated that some improvement in pH stability is realized by replacing silica with alumina, the dissolution of alumina in aqueous solutions at extreme pHs (pH<2 and pH>12), even at room temperature, is well known.

As mentioned previously, in addition to the use of a pH-stable support material, the production of a stable, reversed-phase also requires a process for modifying the support material which results in a stable, hydrophobic surface. Silylation is the most widely used method to derivatize silica particles to produce hydrophobic reversed-phase supports. The silylation of inorganic bodies other than silica (e.g., alumina, titania, zirconia,) has been disclosed in U.S. patent No. 3,956,179. However, it is uncertain whether or not covalent bonds to the support surface are actually formed. In any event, the hydrolytic instability of the siloxane bond is well known, and it is very likely that a Si-O-metal bond will be even more susceptible to aqueous hydrolysis because of the increased polarity of the bond.

An alternate approach to silylation for modifying the surface polarity of inorganic bodies is the sorption of a polymer of desired polarity/functionality onto an $SiO_2$ or $Al_2O_3$ support surface followed by crosslinking of the individual polymer chains to one another to impart additional stability to the coating. Reversed-phase supports prepared in this fashion exhibit much improved pH stability compared to those prepared by silylation. It is important to recognize that the formation of a stable, crosslinked polymer layer on the surface of the support does not reduce the need for a stable, inorganic support, since it may not be possible to cover the entire inorganic surface. Although crosslinking of the polymer may keep it in place even as the underlying inorganic support dissolves, dissolution of the support will undoubtedly lead to a reduction in the mechanical stability of the support. In addition, problems related to increasing column back pressure are known to accompany the dissolution of the inorganic support and its subsequent appearance in the mobile phase and transport through the column and the accompanying instrumentation.

Another problem related to the use of silica-based reversed phase supports is the difficulty encountered in the chromatography of amines and other basic solutes. This problem results from the presence of acidic silanol groups (SiOH) on the silica surface. Basic solutes undergo very strong interactions with these silanol groups which may involve cation exchange or hydrogen bonding, depending on the pH of the mobile phase. Ths problem is exaggerated by the requirement of working in the pH range $2<pH<8$ on silica-based columns, since most amines will be protonated in this pH range and protonated amines can readily bond to the silica surface. One obvious approach to improving the chromatography of amines is to work at hydrogen ion concentrations significantly above the ionization constant of the amines so that they are unprotonated. For aliphatic amines, this normally involves working at a pH greater than 11. However, these pH ranges cannot be employed using silica-based columns.

The presence of the aforementioned acidic silanol groups can also lead to irreversible adsorption of many classes of organic molecules onto silica-based reversed-phase supports, a problem which is well known to those versed in the art. This irreversible adsorption is particularly troublesome in the reversed-phase HPLC of proteins. Ultimately, this adsorption will result in a change in the properties of the support and can lead to its destruction.

Reversed-phase HPLC is finding increased use in the area of bioprocessing because of HPLC's great ability to separate and purify materials. At the preparative scale, there are many unique considerations not applicable at the analytical scale. One such consideration is the need to sterilize a chromatography column prior to its use in the purification of a product intended for bioligical or human use.

It is, therefore, an object of the present invention to produce chromatography column support material which is stable over a wide pH range, and which resists dissolution by aqueous media.

Furthermore, it is an object of the present invention to produce a support material comprising a non-polar surface which can be used for separation by both ion-exchange and reversed-phase processes, wherein the relative contribution of these two processes may be controlled by simple adjustment of mobile phase conditions.

Also, it is the object of the present invention to produce a support material which can be regenerated by freeing it from "irreversibly adsorbed" biological or organic residues by treatment at high pH.

It is another object of the present invention to provide a support material for use in large scale separations, particularly of products generated by biotechnology, for example, by fermentation, that can withstand traditional sterilization techniques involving high pH and heat treatment.

$ZrO_2$ spherules can be prepared by a process consisting of (a) dispersing an aqueous sol containing colloidal $ZrO_2$ particles in a liquid medium extracts the water from the dispersed sol to afford gelled $ZrO_2$ spherules; (b) recovering the gelled spherules from the medium; and (c) heating the gelled spherules to yield solid porous $ZrO_2$ spherules. This process yields porous particles of $ZrO_2$ which are essentially spherical. When formed into a bed, the spherules provide improved mobile phase flow characteristics over those exhibited by irregularly-shaped, jagged-edged or angular particles.

In a preferred embodiment of this process, the colloidal $ZrO_2$ sol is centrifuged, the supernatant liquid decanted and the residue redispersed in an about equal volume of water. This procedure is preferably repeated a plurality of times (2-5X). The redispersed $ZrO_2$ yields spherules having a larger pore diameter and an increased pore volume, when they are formed in accord with the present method.

These particulate spherules can be formed into a bed, and employed as the stationary phase in separations performed via chromatography. Therefore, the spherules can be used as the stationary phase in conventional chromatography columns which have an axial flow path, with or without rigid walls. For example, the $ZrO_2$ spherules can be packed into a column such as a HPLC column, where the packing functions as the stationary phase during HPLC separations which are accomplished by ion exchange and size exclusion processes. The spherules can also be used in columns which have a radial flow path or to form a fluidized bed, with single or multiple stage absorbers. The bed can also be formed of a mass of spherules which are contained in an immobilized enzyme reactor or other type of bioreactor.

The majority of HPLC methodologies involve use of the reverse phase mode, wherein the column-packing material (stationary phase) is non-polar, and the mobile phase is polar. Therefore, the present invention pro-

vides porous $ZrO_2$ spherules coated with a hydrophobic crosslinked polymer coating wherein said coated sperules have a pore size from 2 to 50nm (20-500 A) and a particle diameter range of 0,5-500 µm and are stable to pH 14 in aqueous media. The coated spherules can be prepared by adsorbing a polymerizable monomer or oligomer onto the surface of the spherules and subsequently crosslinking it, eg by reaction of the adsorbed material, with a free radical initiator or by irradiation. The polymeric coating renders the $ZrO_2$ particles hydrophobic without substantially altering any of their desirable physical and mechanical properties.

The coated spherules can also be combined with a suitable binder and used to coat a glass or plastic substrate to form plates for thin-layer chromatography.

Further preferred embodiments are defined in the dependent claims.

As a result of the support material's remarkable stability over a wide pH range, it is useful for the chromatographic separation of compounds at their optimal pHs. For example, the coated material prepared in this fashion can be used for the separation of amines at a variety of pHs and mobile phase conditions such that the separation occurs either by a reversed-phase retention mode, a cation-exchange mode, or some combination of the two. For example, at high pH (pH=12), the amines are unprotonated so that separation occurs entirely by a reversed-phase mode. At low pH in the presence of a low ionic strength phosphate buffer and with an organic solvent-rich mobile phase, the separation occurs via a cation-exchange mode. By adjustement of mobile-phase conditions, selectivity can thus be significantly adjusted.

The $ZrO_2$ spherules of the present invention can also be employed to immobilize bioactive materials for a variety of purposes, including catalysis, analysis, affinity chromatography and synthetic transformations. Bioactive materials can be strongly sorbed onto the exterior and interior surfaces of both the uncoated and the polymer-coated $ZrO_2$ spherules, while retaining a large percentage of their initial bioactivity. Useful biomaterials include proteins such as enzymes and antibodies.

In addition, "irreversibly adsorbed" organic or biological residues can be removed from fouled columns packed with coated or uncoated spherules by flushing the column with a mobile phase at high pH or by injecting pulses of the high pH mobile phase. The term "irreversible adsorption" refers to the very strong tendency which surface-adsorbed proteins, biopolymers and the like exhibit to remain sorbed under normal elution conditions, until the mobile phase conditions are changed sufficiently to desorb them.

Therefore, coated $ZrO_2$ spherules can be prepared which comprise a biologically active material such as an enzyme or a protein such as an immunoglobulin. Upon depletion of the biological activity, the enzyme or other protein can be removed from the spherules by exposing them to an aqueous medium at high pH, e.g., by washing them with a solution of an alkali metal hydroxide. The spherules, stripped of the biological materials, can then be treated with a buffer to return them to a physiological pH, and subsequently reloaded with the same, or a different bioactive material.

The $ZrO_2$ spherules may also be exposed in situ to traditional sterilization conditions, for example, by exposing the packing or the packed column to heat and high pH, without significant degradation. In a further preferred embodiment of the invention, the surface of the coated or uncoated $ZrO_2$ spherules is deactivated or modified by treatment with an effective amount of an organophosphonate prior to or following application of the hydrophobic polymer coating. It is believed that the organophosphonate becomes incorporated into the organic matrix of the polymeric coating.

In the practice of this invention, a portion, or preferably a majority of the initial zirconium oxide ($ZrO_2$) used to form the present spherules is in the sol state; a colloidal dispersion of $ZrO_2$ particles. Such state is clearly a different physical state than is achieved by the simple dispersion of a silica aerogel in water. The latter easily sediments or precipitates because of its relatively larger particle size and lack of stabilizing counter ions in solution. This is in contrast with a colloidal sol where the inorganic particles in an aqueous solution usually are not visible to the naked eye.

Sol particles are submicron in particle size and hence will pass through most common filter papers. Sol particles in water do not aggregate because of a stabilizing electrical charge on the surface which is termed a zeta potential. Once the water is removed, the sol particles interact strongly with one another through hydrogen bonding and Van der Waals forces, to provide aggregated sol particles.

Colloidal dispersions of zirconium oxide suitable for use as the $ZrO_2$ source to prepare the present spherules are commercially available, e.g., as the Nyacol™ series, Nyacol, Inc., Ashland, MA. These dispersions contain about 20 wt-% $ZrO_2$, wherein the $ZrO_2$ particles vary in average diameter, e.g., from 10-250 nm. For example, Nyacol™ Zr 95/20 is an aqueous dispersion containing 20 wt-% $ZrO_2$ of colloidal $ZrO_2$ particles, the majority of which are about 95 nm in diameter.

Non-colloidal $ZrO_2$ sources may be included along with the colloidal $ZrO_2$ particles used to prepare these spherules. Thus, chloride, nitrate, sulphate, acetate, formate or other inorganic or organic salts of zirconium such as the oxysalts and alkoxides may be included with the $ZrO_2$ sol and the mixture used to make spherules. In preferred mixtures, colloidal $ZrO_2$ particles make up the bulk of the total $ZrO_2$ present.

Organic compounds may also be included with the $ZrO_2$ precursors used to prepare the spherules. These organic materials are fugitives which are removed during the firing of the spherules. In particular, water-soluble polymers such as polyvinylpyrolidone, polyethylene glycol, polyethylene oxide, and the like, or latex particles may be included in the liquid mixture used to prepare the spherules. These fugitives may be added to alter the rheology of the precursor solution or the pore structure of the resulting fired spherule.

It is also within the scope of the present invention to include precursors for other metal oxides with the $ZrO_2$ precursors so as to stabilize a particular crystalline phase of $ZrO_2$ or to retard grain growth in the fired spherules. Thus, salts or sols of metals such as yttrium, magnesium, calcium, cerium, aluminum, and the like may be included in levels of from 0-20 mole-%. $ZrO_2$ spherules fired in air or in oxidizing atmospheres which do not contain other oxide additives display either monoclinic, tetragonal or pseudovibic crystal structures when cooled to room temperature. Higher firing temperatures and longer firing times favor the presence of the monoclinic phase. The inclusion of other metal oxides allows the preparation of spherules which possess either monoclinic, tetragonal, or cubic crystalline structures.

Those features of $ZrO_2$ are well known in the art and are discussed in, for example, An Introduction to Zirconia, Magnesium Elektron Ltd., Twickenham, England, (2d ed., Magnesium Elektron Publication No. 113, July 1986).

To prepare the spherical $ZrO_2$, particles, or "spherules," of the present invention, an aqueous sol containing a colloidal dispersion of $ZrO_2$ particles is dispersed in a medium which can extract water from the dispersed sol in the form of droplets. Removal of all or a portion of the water results in gelled solid spherules which consist of aggregated sol particles. One medium which may be used is 2-ethyl-1-hexanol as disclosed in U.S. Patent No. 4,138,336. A preferred medium for safety reasons and ease of processing is peanut oil, which is preferably used at a temperature of 80-100°C. The most preferred medium is a mixture of peanut oil and oleyl alcohol which are combined in a ratio of about 1:1, and used at a temperature of 80-100°C. Oleyl alcohol possesses a higher extraction capacity than peanut oil and mixtures of the two allow the extraction capacity of the medium to be controlled. Depending upon the ratio of sol to forming medium extraction times of from 1-60 minutes can be used to fully gel the $ZrO_2$ particles. The gelled spherules may be conveniently separated from the extracting medium, e.g., by filtration.

Once the $ZrO_2$ particles are condensed into spherules by the above process, thermal treatment at firing temperatures of from 100-1500°C, preferably 400-800°C, is performed. The resulting fired spherules may be from 1-500 $\mu$ in diameter and can possess a surface area of 1-200 $m^2/g$ and pore diameters of from 2 to 50nm (20-500Å). These particles have high mechanical strenght and exceptional stability to aqueous solutions of pHs of 1-14.

The particles may be packed into a HPLC column and used to perform HPLC chromatographic separations by ion exchange and size exclusion mechanism. For a general discussion of HPLC techniques and apparatuses, see remington's Pharmaceutical Sciences, A. Osol, ed., Mack Publishing Co., Easton, PA (16th ed. 1980), at pages 575-576.

The majority of HPLC methodology employs the so-called "reverse phase" mode, i.e., the column-packing material (stationary phase) is non-polar and the eluent (mobile phase) is polar. Therefore, it is a feature of the invention to coat the surface of the $ZrO_2$ spherules with a hydrophobic coating, which is stable to aqueous having a pH of up to 14. Hydrophilic polymer coating can also be applied and crosslinked for modification of the $ZrO_2$ spherules to form an ion exchange support. These hydrophilic polymer coatings are formed from monomers or oligomers which may, for example, comprise polar groups such as sulfonic acids, carboxylic acids, amino groups or quaternary ammonium groups. A preferred method to prepare such a coating comprises sorbing a polymerizable monomer or oligomer onto the surface of the spherules, and crosslinking the monomer or oligomer. See G. Shomberg, LC-GC, 6, 36 (1988).

## A. Polymerizable Monomers or Oligomers

A wide variety of crosslinkable organic materials, which may be monomers, oligomers or polymers, can be employed to coat the porous $ZrO_2$ spherules. For example, such materials include polybutadiene, polystyrene, polyacrylates, polyvinylpyrrolidone (PVP), polyorganosiloxanes, polyethylene, polyethylene imine, polyaspartic acid and multifunctional silanes.

A preferred material for the preparation of a reversed phase support material is an oligomer of polybutadiene. A preferred material for modification of the $ZrO_2$ spherules to form a cation ion exchange support is polyaspartic acid. A preferred material for construction of a support suitable for aqueous steric exclusion chromatography is a tri- or di-alkoxy-, gamma-glycidoxy silane.

5

## B. Crosslinking Agents

Any of the common free radical sources including organic peroxides such as dicumyl peroxide, benzoyl peroxide or diazo compounds such as 2,2'-azobisisobutyronitrile (AIBN) may be employed as cross-linking agents in the practice of the present invention. Useful commercially available peroxyesters include the alkylesters of peroxycarboxylic acids, the alkylesters of monoperoxydicarboxylic acids, the dialkylesters of diperoxydicarboxylic acids, the alkylesters of monoperoxycarbonic acids and the alkylene diesters of peroxycarboxylic acids. These peroxyesters include t-butyl peroctoate, t-butyl perbenzoate, t-butyl peroxyneodecanoate and t-butyl peroxymaleic acid. These compounds are commercially available from Pennwalt Chemicals, Buffalo, NY. The amount of any free radical initiator required to catalyze the polymerization reaction will vary depending upon the molecular weight of the initiator and its thermal stability. Oligomers may also be polymerized by thermal treatment, by irradiation with UV light or gamma rays or by exposure to high energy electrons.

## C. Coating/Crosslinking Process

Zirconium oxide may be modified in different ways to achieve materials with a light, intermediate or heavy carbon load. Preferably, the $ZrO_2$ spherules are first surface-hydrated and then dried in vacuo. Depending on the load desired, the dried $ZrO_2$ spherules are added to 15-50 ml of a pentane solution containing from 5-250 mg of an oligomer, such as polybutadiene, per gram of $ZrO_2$ spherules. The resultant slurry is placed in an ultrasonic bath and a vacuum applied in order to degas the particles and to insure that the oligomer solution has infiltrated substantially all of the pores. A free radical initiator, such as dicumyl peroxide, is then added at a level of 2-20% (w/w) relative to the amount of polymer used. Solvent is then removed either by evaporation or by filtration, again depending on the desired carbon load. The treated $ZrO_2$ spherules are then heated to 60-70°C under vacuum (1.33-2.67kPa) (10-20mm Hg) for 12hrs to remove any remaining solvent. The cross-linking reaction is then carried out by heating the coated $ZrO_2$ spherules in a tube furnace at 175-200°C for 2-4 hours under a flow of nitrogen.

The resultant coated spherules are then packed into 5 cm x 0.46 cm HPLC columns by dry packing or stirred upward slurry packing, depending on their particle size.

Mixed-mode chromatography of amines can be performed in aqueous/organic mobile phases at various pHs containing different amounts of organic solvent, phosphate buffer and neutral salt for ionic strength adjustment.

A column "fouled" by repeated injections of large amounts of material, to the point that a marked change in characteristics is observed, can be stripped of irreversibly adsorbed material. The original column performance can be restored by pulsing the column with 100 μl injection of 1 M NaOH or by flushing the column for about 0.5-10 hrs with aqueous alkali metal hydroxide, i.e., with a 0.1 M NaOH solution.

The stability of the polymer-coated $ZrO_2$ spherules or uncoated $ZrO_2$ spherules to sterilizing conditions can be demonstrated by heating a previously characterized column to 100°C while pumping a 1 M NaOH solution through it for 12-24 hrs. Recharacterization of the column demonstrates that no significant change in column properties or decreased retention of a non-polar substance has taken place.

The surface of uncoated or polymer-coated $ZrO_2$ spherules can be easily and dramatically modified in a chromatographically-beneficial way by treatment with aqueous inorganic phosphate solutions. The interaction between polymer-coated $ZrO_2$ and phosphate produces a mixed mode stationary phase exhibiting both cation-exchange and reversed-phase properties. This allows one to adjust the selectivity of the present support material with respect to a group of basic solutes by appropriate adjustement of mobile phase pH, ionic strenght, and reversed-phase eluting strength (i.e., volume fraction of the adjuvant organic solvent).

Useful aqueous inorganic phosphate solutions include 0.01-1.0 M solutions of $H_3PO_4$ or of alkali metal phosphate salts, e.g., orthophosphates, pyrophosphates, metaphosphates, tripolyphosphates and the like.

For some applications, it is desirable to further deactivate or modify the surface of the uncoated or polymer-coated $ZrO_2$ spherules. This can be used to enhance the ability of the spherules to separate polar compounds such as amines and carboxylic acids, or to modify retention. This deactivation can be accomplished by treating the uncoated $ZrO_2$ spherules with an organophosphorus compound in a suitable solvent for the organophosphorus compound. Useful organophosphorus compounds include allylphosphonate, octyl phosphonate, phenyl phosphonic acid, napthyl phosphonic acid, phenyl phosphinic acid, phenylphosphoric acid and diallyl phosphorate. Preferred organophosphorus compounds include the unsaturated organophosphonic acids and the water-soluble salts thereof.

Useful solvents for the organophosphorus compound include aqueous alcohol, e.g., a solution of water and a ($C_1$-$C_5$)alkanol. The $ZrO_2$ spherules are preferably coated by agitating the spherules in a solution of the organophosphorus compound so that the weight ratio of the organophosphorus compound to spherules is 0.25-1:1. The treated particles are then separated from the treating solution, dried and the crosslinked polymeric coating applied as discussed hereinabove.

The invention will be further described by reference to the following detailed examples.

## Preparation Example 1

Peanut oil (3 liters) was placed in a 4 liter beaker and heated to 90°C. A mechanical agitator was inserted and the peanut oil was vigorously stirred. One hundred grams of Nyacol™ Zr 95/20, a colloidal $ZrO_2$ manufactured by Nyacol, Inc. and containing 20 wt-% of $ZrO_2$, primarily as about 95 nm particles, was sprayed into the peanut oil through an aerosol atomizer. After approximately 30 minutes, the batch was filtered through a No. 54 Whatman filter. Approximately 17 g of solids were recovered, which were predominately spherules having a diameter of < 30 μ.

## Preparation Example 2

Peanut oil (600 g) and 600 g of oleyl alcohol were mixed and heated to about 90°C. Under vigorous agitation, 100 g of Nyacol™ Zr 95/20 was sprayed into the peanut oil/oleyl alcohol mixture as described Example 1. After 30 minutes, the batch was filtered and the particles collected. The particles were predominantly (ca: 70%) spherules having a diameter of < 50 μ.

Spherules prepared as described in Examples 1 and 2 were thermally treated at a series of temperatures and the surface area, average pore diameter and pore volume were measured by nitrogen adsorption isotherm on a Quantasorb surface area analyzer. These results are summarized in Table I, below.

## Table I

| Firing Temp (°C)* | Surface Area (m²/g) | Average Pore Diameter nm [Å] | Pore Volume (%) |
|---|---|---|---|
| 400 | 142 | 4.2 (42) | 47 |
| 500 | 92 | 71. (71) | 50 |
| 600 | 34 | 11.0 (110) | 36 |
| 800 | 17 | 20.5 (205) | 34 |
| 900 | 14 | 22.0 (220) | 31 |

* 6 hrs

The data summarized on Table I show that it is possible to increase the average pore diameter by increasing the firing temperature from 400 to 900°C. The surface area and pore volume decrease with increasing firing temperature. Chromatographic activity of the $ZrO_2$ spherules is determined by the parameters of surface area, average pore diameter and pore volume. Accordingly, the appropriate firing temperature is selected.

## Preparation Example 3

The procedure of Preparation Example 2 was used to prepare spherules using Nyacol™ Zr 50/20, a colloidal $ZrO_2$ supplied by Nyacol, Inc. (50 nm $ZrO_2$ colloid size) as the $ZrO_2$ source.

## Preparation Example 4

The procedure of Preparation Example 2 was used to prepare spherules using Nyacol™ Zr 150/20, a colloidal $ZrO_2$ supplied by Nyacol, Inc. (150 nm $ZrO_2$ colloid size) as the $ZrO_2$ source.

Table II summarizes the surface area, average pore diameter and pore volume of spherules prepared as per Preparation Example 2-4 and fired at 600°C for 6 hrs.

## Table II

| ZrO2 Source* | ZrO2 Colloid size (nm) | Surface Area (m²/g) | Average Pore Diameter nm [Å] | Pore Volume (%) |
|---|---|---|---|---|
| Zr 50/20 | 50 | 33 | 9.2 (92) | 31 |
| Zr 95/20 | 95 | 34 | 11.0 (110) | 36 |
| Zr 150/20 | 150 | 40 | 14.7 (147) | 45 |

*Nyacol™ series.

The data summarized in Table II show that it is possible to control the average pore diameter of the fired spherules by appropriate selection of the colloid size of the $ZrO_2$ source. Larger colloids produce fired spherules with larger pore diameters and pore volumes.

### Preparation Example 5

Nyacol™ Zr 95/20 colloidal $ZrO_2$ was placed in a laboratory centrifuge and sedimented. The supernatant was decanted and discarded. The sedimented $ZrO_2$ was redispersed in an equal volume of distilled water. Spherules were prepared from this centrifuged sol following the procedures of Preparation Example 2

### Preparation Example 6

The centrifugation procedure of Preparation Example 5 was performed and the redispersed sol was subsequently recentrifuged to sediment, the supernatant decanted off and the $ZrO_2$ redispersed. Spherules were prepared from this doubly centrifuged sol following the procedure of Preparation Example 2.

### Preparation Example 7

The double centrifugation procedure used in Preparation Example 6 was performed and the redispersed sol was subsequently recentrifuged to sediment, the supernatant decanted, and the $ZrO_2$ redispersed. Spherules were prepared from this triply centrifuged sol following Preparation Example 2.

Table III summarizes the surface area, pore diameter and pore volume of spherules prepared as per Preparation Examples 2, 5, 6 and 7 and heated to 600°C for 6 hrs.

## Table III

| ZrO2 Source* | Surface Area (m²/g) | Average Pore Diameter nm [Å] | Pore Volume (%) |
|---|---|---|---|
| Zr 95/20 | 34 | 11.0 (110) | 36 |
| Zr 95/20 cent.(1x) | 50 | 16.2 (162) | 55 |
| Zr 95/20 cent.(2x) | 52 | 23.5 (235) | 62 |
| Zr 95/20 cent.(3x) | 46 | 25.0 (250) | 62 |

*Nyacol™ Zr series.

EP 0 331 283 B1

Centrifugation, removal of the supernatant, and redispersion of the colloidal $ZrO_2$ starting material results in increases in the average pore diameter, pore volume and surface area of fired spherules. This increase is believed to result from the removal of small (5-10 nm) colloidal $ZrO_2$ particles which are known to be present in the Nyacol™ Zr series sols as a minor component. Many of these smaller $ZrO_2$ particles remain suspended during centrifugation and are removed when the supernatant is discarded prior to redispersion of the larger sedimented $ZrO_2$ particles. If present, these small $ZrO_2$ particles are believed to increase the packing density of the spherules by filling the interstices between larger $ZrO_2$ particles and therefore decreasing the average pore diameter, pore volume and surface area of the fired spherules.

It is also possible that sedimentation by centrifugation may result in agglomeration of the colloidal $ZrO_2$ particles into aggregates which pack together in a more open structure (effectively behaving as larger particles) than unaggregated particles.

Regardless of mechanism, the centrifugation treatment described in Preparation Examples 5-7 provide a method of preparing spherules with increased average pore diameter, pore volume and surface area relative to spherules prepared from untreated colloidal $ZrO_2$ sols.

The following example demonstrates the use of the unmodified $ZrO_2$ spherules prepared as described above in the chromatographic separation of proteins.

Example 1

A stationary phase suitable for anion exchange chromatography was prepared by adsorption of polyethyleneimine [Polysciences, Inc., Warrington, PA] and subsequent crosslinking with 1,4-butanediol digylcidyl ether (95%, Aldrich Chemical Co., Milwaukee, WI); by the method of Regnier et al., J. Chromatog., 185, 375 (1979); 318, 157 (1985), 359, 121 (1986).

The anion exchange capacity for adsorption of picric acid was determined to be 230 μmoles/g of modified $ZrO_2$. This substrate was used to separate ovalbumin from BSA. The column was operated with a gradient of 10 mM Tris buffer at pH 7.5 to 10 mM Tris at pH 7.5 with 0.5 M NaCl over 20 min, followed by an additional 10 min of isocratic operation at pH 7.5 with 0.5 M NaCl. The flow rate was 1 ml/min. The retention times were 9.75 (ovalbumin) and 22.8 min (BSA).

The following examples demonstrate the use of the $ZrO_2$ spherules to immobilize proteins.

**Comparative Example I**

$ZrO_2$ spherules with a diameter of approximately 30 μ and a surface area of 50 m²/g and an average pore diameter of 12.4 nm (124 Å) were used. Mouse anti-human IgE antibody was purified and radioiodinated. ($I^{125}$) by the method of S. M. Burchiel et al., J. Immunol. Meth., 69, 33 (1984); K. L. Holmes et al., PNAS USA, 82, 7706 (1985), and diluted with unlabelled antibody to yield a specific radioactivity of 5,000 cpm/μg. A portion of 250 μl of antibody (250 μg/ml in 5 mM Tris, pH 8.0) was added to tubes containing 10 mg of spherules. The mixture was briefly evacuated, then rocked at ambient temperature for the appropriate time, 5-120 min., with three replicates for each time point. The tubes were centrifuged and rinsed twice with 1 ml of buffer. The spherules were transferred to a fresh tube along with 2 ml of buffer, the buffer removed and the radioactivity of each tube was determined in a Packard Model 5230 gamma scintillation counter. The amount of bound protein in ng, converted from cpm, is shown in Table V.

Table V

| Time (Min) | Antibody Bound/mg Spherules |
|---|---|
| 10 | 54 ng |
| 20 | 66 ng |
| 30 | 72 ng |
| 60 | 69 ng |
| 120 | 62 ng |

**Comparative Example 2**

Using the same materials and techniques described in Comparative Example I the extent of binding of an-

9

tibody in 2 hr incubations as a function of its concentration (1-250 µg/ml) was determined. The averages of three replicates show a saturation (table VI)O. Double-reciprocal analysis of these data extrapolate to 100 ug antibody bound per g spherule at saturation.

Table VI

| Conc. Protein (µg/ml) | Antibody Bound/mg Spherules |
|---|---|
| 1 | 1.5 ng |
| 5 | 7.5 ng |
| 10 | 14.0 ng |
| 50 | 38.0 ng |
| 250 | 62.0 ng |

## Comparative Example 3

### A. Trypsin Immobilization.

Solutions (2 mg/ml) of trypsin, a 24 KDa proteolytic enzyme and bovine serum albumin (BSA), a 67 KDa protein, were bound to 70 mg of the $ZrO_2$ spherules (average pore diameter 10 nm (100 Å) surface area of 30 $m^2/g$ in 5 mM tris, pH 8.0 by agitating the degassed spherules in 1.0 ml of buffer for 17.5 hr. Trypsin (15.3 mg) and < 0.2 mg (BSA) bound per g of spherule, a proportion which might be expected from their relative sizes and the size of the pores.

Trypsin was assayed using the thioesterase assay disclosed by P. L. Coleman et al., Meth. Enzymol., 80, 408 (1981). The bound spherules were suspended in 1 ml of buffer and a 5 µl aliquot was added to a tube containing 1.0 ml of substrate. After 2.5 min of continuous shaking, a citrate-soybean trypsin inhibitor (STI) solution was added to quench the reaction. It was rapidly centrifuged and the supernatant removed for determination of the absorbance (A) at 412 nm. Assays were performed on trypsin spherules, BSA spherules and the supernatant from trypsin spherules. The assay was also performed with the trypsin inhibitor in the substrate solution to determine whether it was able to inhibit the bound trypsin. The results of these assays are summarized on Table VII, below.

Table VII

| Sample | Trypsin Activity (A at 412 nm) | |
|---|---|---|
| | -STI | +STI |
| Trypsin spherules | 2.36 | 1.79 |
| BSA spherules | 0.10 | 0.13 |
| Trypsin supernatant | 0.19 | 0.12 |

The results (Table VII) indicate that about 75% of the bound activity is unavailable to STI, even though STI is smaller than trypsin. In addition, only 4% of the activity is attributable to unbound trypsin, a surprisingly low value given the inefficient batch washing method which was used.

Calculations based on these observations demonstrated several unexpected results. For example, 15 mg of trypsin/g $ZrO_2$ corresponds to 51 mg/ml using 3.3 g/ml as the density of the spherules. This corresponds to a trypsin concentration of 2 mM in the column. A check on this may be made by estimating the expected A at 412 nm for the assay. In these assays, the spherule-bound enzyme was 0.21 µM, the kcat for the substrate is 75/sec [G. D. J. Green et al., Anal. Biochem., 93, 223 (1979)] and the extinction coefficient is 14,100, yielding an estimated 3.3 absorbance change, which compares favorably with the 2.4 observed. Since chromogen was present in amount sufficient to give only 0.28 nm (2.8 A) at 412 nm, it is safe to assume that nearly all of the bound trypsin is active. Thus, an extraordinary amount of protein is bound and retains its enzymic activity.

## B. Chymotrypsinogen-Chymopapain-BSA Immobilization.

The procedure of Preparation Example 7 was employed to prepare $ZrO_2$ spherules having 24 nm (240 Å) pores and a surface area of 27 $m^2$/g. Small columns were poured, each containing about 1.0 g of spherules, and were equilibrated with either 20 mM tris-chloride buffer (pH 8.0) or 50 mM sodium acetate buffer (pH 4.5). Chymotrypsinogen (24.5 kDa) and chymopapain (32 kDa) were dissolved in the tris buffer and BSA was dissolved in the acetate buffer. Protein-containing solution was continuously added to the column until the 280 nm absorbance of the eluate equalled that of the starting solution. Unbound protein was rinsed from the column, and the amount of bound protein was calculated from the difference between that added and that recovered in the eluate.

Chymotrypsinogen and chymopapain bound at 76.9 mg and 24.5 mg of protein/g of spherules at pH 8.0, respectively, and 64 mg of BSA bound per gram of spherules at pH 7.5. Converting these values into binding densities per ml of column volume yields 254, 81 and 211 mg/ml of protein, respectively.

The fact that at acidic pH, albumin binds to a greater extent than does the smaller chymopapain, and almost to the extent as the even smaller chymotrypsinogen suggests that the latter enzymes would bind to even greater densities at lower pH, i.e., below their pla.

## Example 2

### Polymer Adsorption/Crosslinking.

Preparation A - Heavily loaded $ZrO_2$: A solution of 0.55 g of polybutadiene (Aldrich Chemical Co., Milwaukee, WI, m.w. 4500, Cat. No. 20-050-6) in 50 ml of pentane was added to 3.5 g of $ZrO_2$ spherules prepared as described in Preparation Example 2 (fired at 600°C for 6 hrs; particle size = 20-45 μm(microns) which had been boiled in $CO_2$-free water to fully hydrate the surface and then dried at 125°C. The slurry was placed in an ultrasonic bath and a water aspirator vacuum applied. Dicumyl peroxide (DCP) (0.01 g) was then added and the slurry was again placed in an ultrasonic bath and a vacuum applied. The pentane was removed in vacuo and the material dried at 70°C under vacuum. The material was then heated in a tube furnace at 200°C for 2 hrs and then washed successively with pentane, toluene, methylene chloride, tetrahydrofuran, methanol and 0.1 M sodium hydroxide. Elemental analysis of the coated spherules showed a carbon load of 7.7%. A duplicate sample was prepared in an identical fashion and had a carbon load of 7.5%. Because of the extremely heavy load of polybutadiene, the specific surface area of the porous spherules, as determined by a BET measurement, decreased from 50.4 to 4 $m^2$/gm.

Preparation B - Lightly loaded $ZrO_2$: 35 ml of solution of 0.09 g of polybutadiene in pentane was added to 3.5 g of $ZrO_2$ spherules and the resultant slurry was placed in an ultrasonic bath and a water aspirator vacuum applied. Pentane (10 ml) containing 0.002 g of DCP was then added and the slurry was again placed in an ultrasonic bath and a vacuum applied. The slurry was then shaven for one hr and the supernatant removed by filtration. The material was then washed as described in preparation A. Elemental analysis of the coated spherules showed 0.84% carbon, while the BET results showed a specific surface area of 38.7 $m^2$/gm. The decrease in specific surface area from 50.4 to 38.7 $m^2$gm is similar to the reduction in surface area which occurs upon silylation of typical inorganic supports.

Preparation C - Intermediate load: A solution of 0.27 g of PBD in 50 ml pentane was added to 3.0 g of $ZrO_2$ spherules (mean particle diameter 3.5 microns). The slurry was placed in an ultrasonic bath and a vacuum applied. 5.2 mg of DCP in 10 ml of pentane were then added. The methodology of preparation A was then followed. Elemental analysis showed 2.7% carbon.

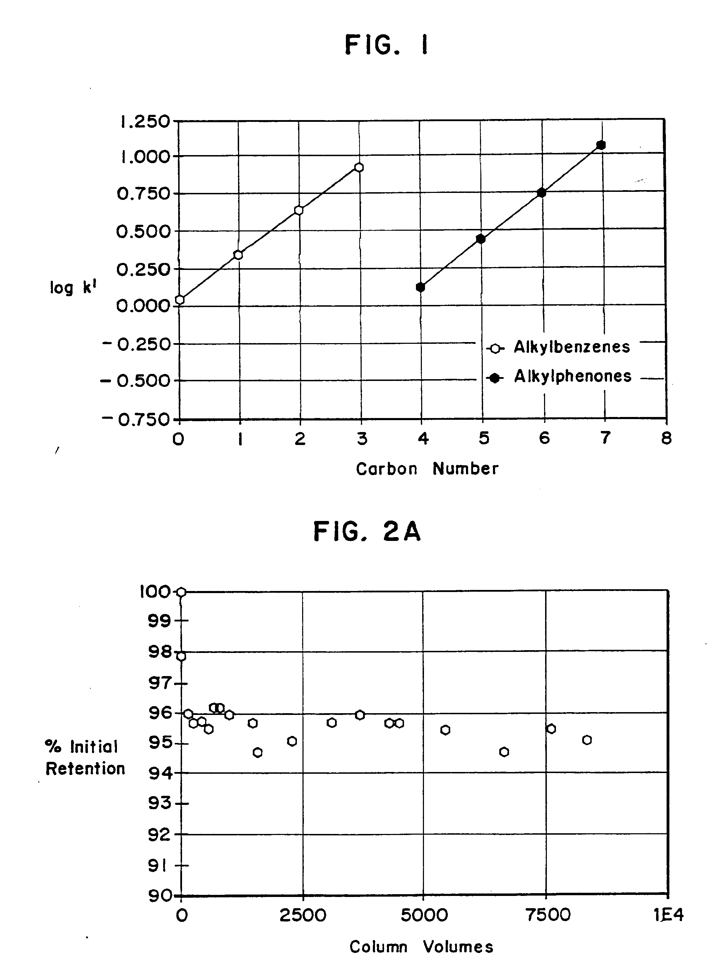

It is clear from the results of carbon analysis that carbon had been deposited on the surface of the $ZrO_2$ spherules. Figure 1 further demonstrates the reversed-phase nature of the polymer-modified $ZrO_2$ spherules as exhibited by a 5 cm x 0.46 cm column packed using preparation C. The linearity of the log k' (capacity factor) vs. carbon number plot for the members of a homologous series of alkylphenones is clearly indicative of a reversed-phase retention mechanism.

## Example 3

A mixed cation-exchange/reversed phase support was prepared by treating a material prepared as described in Example 2, Preparation C with a 100 mM aqueous $H_3PO_4$ solution at pH 3 for about one hour. The retention data given in Table VIII show distinct changes in selectivity as a function of pH, volume fraction organic solvent and mobile phase ionic strength.

Table VIII

Selectivity Factor*

| Solute | A | B | C | D | E |
|---|---|---|---|---|---|
| Butyl Benzene | 5.02 | 5.18 | 4.86 | 6.89 | 6.94 |
| Lidocaine | 0.28 | 0.074 | 0.1 | 0.44 | 0.32 |
| Quinine | 2.9 | 0.39 | 0.22 | 5.17 | 0.6 |
| Nortriptyline | 68.0 | 2.61 | 2.07 | 99.2 | 3.38 |
| Amitriptyline | 15.6 | 3.29 | 3.45 | 33.7 | 5.69 |

*Selectivity Factor = [k' (solute)]/[k'(toluene)] Conditions, and capacity factors of toluene are given below:

A = 60% MeOH/40% 10 mM PO4 at pH 7; k' (toluene) = 0.57

B = 60% MeOH/40% 10 mM PO4 at pH 7 with 0.5 M NaCl k' (toluene) = 0.54

C = 60% MeOH/40% 10 mM PO4 at pH 12; k' (toluene) = 0.58

D = 50% MeOH/50% 10 mM PO4 at pH 7; k'(toluene) = 1.2

E = 50% MeOH/50% 10 mM PO4 at pH 12; k'(toluene) = 1.2

Separations at high pH (above the pKa of the amines) are dominated by a reversed-phase retention mechanism as are separations at lower pH in high ionic strength mobile phase. Conversely, separations at low pH in a low ionic strength environment are controlled primarily by cation-exchange processes. In addition to the ability to alter selectivity in several ways, the subject material also exhibits dramatic improvement in terms of the peak symmetry of amine solutes relative to silica.

**Example 4**

The pH stability of the material of Example 2 , preparation A, has been demonstrated in chromatographic experiments at high pH and elevated temperature by monitoring the retention of test solutes and by measurement of the amount of carbon on the support before and after prolonged exposure to high pH. These experiments were carried out under the following chromatographic conditions: Mobile phase A: 0.1 M $CO_2$-free NaOH; Mobile phase B: Methanol; Flow Rate: 1 ml/min; Oven Temp: 50°C.

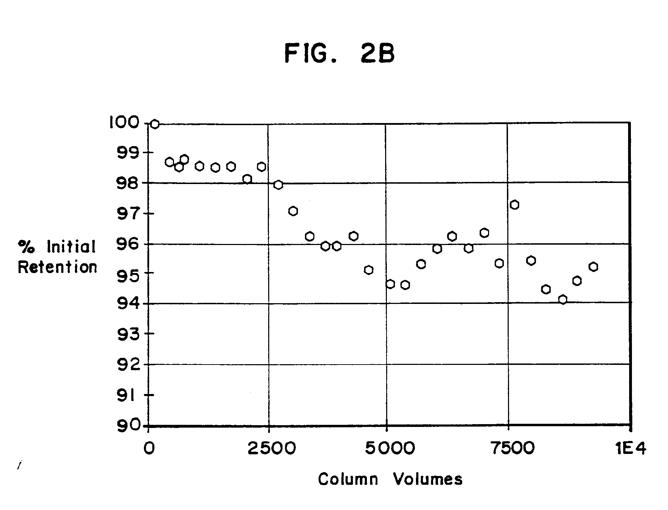

The retention of two test solutes in a mobile phase of 50% B/50% A as a function of the number of column volumes of mobile phase flushed through the column is shown in Figure 2A. Note that the initial decrease in retention reflects the equilibration of the column to the elevated temperature and not a loss in bonded phase. The evaluation was repeated on the lightly loaded material (Preparation B); the retention data on this material is shown in Figure 2B. Once again, there is an initial decrease in retention associated with column equilibration. There is also a slight decrease in retention at approximately 15 hours which accompanied a change in the lot of mobile phase; this change does not reflect a significant drop in carbon load.

It is believed that the above evaluations represent the most challenging test of pH stability which has been reported for any reversed-phase material and it is also believed that the data clearly show that the spherules of Example 2 , Preparations A and B, are essentially stable under these conditions.

## Example 5

$ZrO_2$ spherules prepared by the procedure of Preparation
Example 2 (3.4 g, surface area: 60 m²/g; pore diameter: 9.5 nm (95 Å) were treated with a solution of 1.6 g of allylphosphonate in 50 ml of 95/5 (v/v) methanol/water. After "ultrasonicating" under vacuum and shaking for one hr, the supernatant was removed by filtration and the phosphonate-treated $ZrO_2$ was dried at 70°C for 12 hrs. The material was then modified with PBD according to Example 2 Preparation C. In this manner, the residual $ZrO_2$ surface was deactivated as is clearly shown by the data in Table IX. Note that carboxylic acids are not eluted on the non-phosphonated $ZrO_2$ material but are eluted on the phosphonated material.

### Table IX

| Solute | k'(untreated) | k'(treated) |
|---|---|---|
| Toluene | 0.46 | 0.49 |
| Benzoic Acid | not eluted | 6.1 |

## Example 6

Several 100 µl injections of cytochrome C were made on a column packed with material prepared as described in Example 2 Preparation C. The retention of cytochrome C on this material decreased due to "irreversible" adsorption of protein upon each injection.

The column was then "pulsed" with 5, 100 µl injections of 1 M NaOH in order to strip the "irreversibly adsorbed" cytochrome C. The effect of the pulses is to strip the column of adsorbed protein such that the original retention characteristics can be regenerated.

## Example 7

The ability of the subject matter to withstand sterilizing conditions was demonstrated by taking a material prepared as described in Example 2 preparation C, and evaluating its chromatographic characteristics, by exposing it to 1 M NaOH at 100°C for 1 hr and then reevaluating the chromatographic properties. As shown by the data in Table X, there was no decrease in retention of nonpolar substances upon challenging the packing in this fashion.

### Table X

| Solute | k' Before Treatment | k' After Treatment |
|---|---|---|
| Benzene | 1.36 | 1.47 |
| Toluene | 2.68 | 3.01 |
| Ethyl Benzene | 4.83 | 5.57 |
| Propyl Benzene | 9.21 | 10.86 |

A second column (ES Industries, Marlton, NJ) which uses an alumina support modified by the method of G. Shomberg, LC-GC, 6, 36 (1988), was challenged with a mobile phase of 1M NaOH, which was collected in two fractions. The first corresponded to an elution time of 1 hr and the second to an additional elution of 2.25 hrs.

The elements were analyzed via an inductively coupled plasma spectrometer. The concentration of aluminum- in the eluent from the second column corresponded to the dissolution of a total of 10% of the mass of the alumina in the column.

In marked contrast, zirconium was absent at a level of detectability of 0.03 µg/ml. Even if Zr was present at the detection limit, this would correspond to loss of less than 0.001% of the mass of $ZrO_2$ on the test column.

## Claims

1. Porous $ZrO_2$ spherules coated with a hydrophobic crosslinked polymer coating wherein said coated spherules have a pore size from 2 to 50nm (20-500 A) and a particle diameter range of 0.5-500 µm and are stable to pH 14 in aqueous media.

2. Spherules as claimed in claim 1 wherein the polymer coating comprises polybutadiene.

3. Spherules as claimed in claim 1 or 2 wherein the surface of the $ZrO_2$ spherules is modified by treatment with inorganic phosphate to impart both cation-exchange and hydrophobic characteristics to said spherules.

4. Spherules as claimed in claim 3 wherein the inorganic phosphate is $H_3PO_4$.

5. Spherules as claimed in claim 1 or 2 whose surface is deactivated by treatment with an organophosphorus compound.

6. Spherules as claimed in claim 5 wherein said orgonophosphorus compound is an unsaturated organophosphonate or organophosphonic acid.

7. Spherules as claimed in claim 6 wherein the organophosphonate is allylphosphonate.

8. Spherules as claimed in any of claims 5, 6 and 7 wherein said surface has been treated with said organophosphorus compound prior to application of the hydrophobic polymer coating.

9. Spherules as claimed in any of claims 5, 6 and 7 wherein said surface has been treated with said organophosphorus compound after application of the hydrophobic polymer coating.

10. A thin layer chromatography plate comprising a substrate coated with a mixture of a binder and a particulate support material comprising spherules as claimed in any of claims 1, 3, 6 and 7.

## Patentansprüche

1. Poröse $ZrO_2$-Kügelchen, die mit einem hydrophoben vernetzten Polymerüberzug beschichtet sind, wobei die beschichteten Kügelchen eine Porengröße von 2 bis 50 nm (20 bis 500 Å) und einen Teilchendurchmesser im Bereich von 0,5 bis 500 µm aufweisen und in wässrigen Medien bis zu pH 14 stabil sind.

2. Kügelchen nach Anspruch 1, bei denen die Polymerschicht Polybutadien umfaßt.

3. Kügelchen nach Anspruch 1 oder 2, bei denen die Oberfläche der $ZrO_2$-Kügelchen durch Behandlung mit anorganischem Phosphat modifiziert wird, um den Kügelchen sowohl Kationenaustausch- als auch hydrophobe Eigenschaften zu verleihen.

4. Kügelchen nach Anspruch 3, bei denen das anorganische Phosphat $H_3PO_4$ ist.

5. Kügelchen nach Anspruch 1 oder 2, bei denen die Oberfläche durch Behandlung mit einer Organophosphorverbindung deaktiviert wurde.

6. Kügelchen nach Anspruch 5, bei denen die Organophosphorverbindung ein ungesättigtes Organophosphonat oder Organophosphonsäure ist.

7. Kügelchen nach Anspruch 6, bei denen das Organophosphonat Allylphosphonat ist.

8. Kügelchen nach einem der Ansprüche 5, 6 und 7, bei denen die Oberfläche vor der Aufbringung des hydrophoben polymeren Überzugs mit der Organophosphorverbindung behandelt wurde.

9. Kügelchen nach einem der Ansprüche 5, 6 und 7, bei denen die Oberfläche nach der Aufbringung des hydrophoben polymeren Überzugs mit der Organophosphorverbindung behandelt wurde.

10. Dünnschichtchromatographie-Platte, umfassend ein mit einer Mischung aus einem Bindemittel und einem feinverteilten Trägermaterial beschichtetes Substrat, welches Kügelchen nach einem der Ansprüche 1, 3, 6 und 7 aufweist.


**Revendications**

1. Sphérules poreuses de $ZrO_2$ revêtues d'un revêtement polymère réticulé hydrophobe où lesdites sphérules ont une dimension de pore de 5 à 50 nm (20-500 A) et diamètre particulaire compris dans l'intervalle de 0,5 à 500 micromètres, et sont stables jusqu'à pH 14 en milieu aqueux.

2. Sphérules suivant la revendication 1, dans lesquelles le revêtement polymère contient du polybutadiène.

3. Sphérules suivant la revendication 1 ou 2, dans lesquelles la surface des sphérules de $ZrO_2$ est modifiée par traitement avec un phosphate minéral pour fournir à la fois des propriétés d'échange de cations et hydrophobes aux dites sphérules.

4. Sphérules suivant la revendication 3, dans lesquelles le phosphate minéral est $H_3PO_4$.

5. Sphérules suivant la revendication 1 ou 2, où la surface est désactivée par traitement avec un composé organophosphoreux.

6. Sphérules suivant la revendication 5, dans lesquelles ledit composé organophosphoreux est un acide organophosphonique ou un organophosphonate insaturé.

7. Sphérules suivant la revendication 6, dans lesquelles le organophosphonate est un allylphosphonate.

8. Sphérules suivant l'une quelconque des revendications 5, 6 et 7, dans lesquelles ladite surface a été traitée avec ledit composé organophosphoreux avant application du revêtement polymère hydrophobe.

9. Sphérules suivant l'une quelconque des revendications 5, 6 et 7, dans lesquelles ladite surface a été traitée avec ledit composé organophosphoreux après application du revêtement polymère hydrophobe.

10. Plaque pour chromatographie sur couche mince comprenant un substrat revêtu d'un mélange d'un liant et d'un matériau support particulaire comprenant des sphérules telles revendiquées dans l'une quelconque des revendications 1, 3, 6 et 7.

## FIG. I

## FIG. 2A

# FIG. 2B